Europäisches Patentamt

**European Patent Office**

**Office européen des brevets**

(19)

(11) Numéro de publication: **0 033 668**
**B1**

(12) ## FASCICULE DE BREVET EUROPEEN

(45) Date de publication du fascicule du brevet:
**15.06.83**

(51) Int. Cl.³: **B 05 B 7/16,** A 61 M 11/06,
F 24 F 6/14, A 61 L 9/14

(21) Numéro de dépôt: **81400007.1**

(22) Date de dépôt: **06.01.81**

(54) **Procédé et appareil de production d'aérosols à particules sèches et ou de brouillard humide.**

(30) Priorité: **08.01.80 FR 8000264**

(43) Date de publication de la demande:
**12.08.81 Bulletin 81/32**

(45) Mention de la délivrance du brevet:
**15.06.83 Bulletin 83/24**

(84) Etats contractants désignés:
**BE CH DE GB IT LI NL**

(56) Documents cités:
**DE-A-2 711 060**
**DE-B-1 046 264**
**DE-B-1 220 559**
**DE-C-302 161**

(73) Titulaire: **DAULANGE, Jacques, Zone Industrielle Boîte Postale No 9, F-24120 Terrasson (FR)**

(72) Inventeur: **DAULANGE, Jacques, Zone Industrielle Boîte Postale No 9, F-24120 Terrasson (FR)**

(74) Mandataire: **Loyer, Bertrand et al, Cabinet Pierre Loyer 18, rue de Mogador, F-75009 Paris (FR)**

BUNDESDRUCKEREI BERLIN

## Procédé et appareil de production d'aérosols à particules
### sèches et/ou de brouillards humides

L'invention se rapporte à un procédé ainsi qu'à un appareil de production d'aérosols à particules sèches et ou de brouillard humide de liquides et notamment de liquides désinfectants, vétérinaires ou médicamenteux.

Les besoins sans cesse croissants de production soit d'aérosols à particules sèches soit de pulvérisations humides notamment dans les exploitations d'élevage et agricoles rendent nécessaire l'emploi d'appareils capables de fournir économiquement et rapidement des quantités appréciables de substances sous des formes bien définies et constantes.

On connaît depuis longtemps des appareils pulvérisateurs ainsi que des appareils de production d'aérosols. Cependant, ces appareils sont plus ou moins bien adaptés à un emploi précis et ne permettent pas de faire varier dans des proportions appréciables les conditions de pulvérisation et de production d'aérosols à particules sèches.

On connaît encore des appareils permettant de produire des aérosols à particules sèches obtenues par caléfaction sur une plaque chauffante.

Cependant ces appareils pulvérisaient les particules de liquides provenant d'un ajutage horizontal par l'émission d'un flux d'air dirigé perpendiculairement à celui-ci et vers le bas en direction d'une plaque chauffante comme par exemple les appareils décrits aux brevets DE-C-302 161 (S. Feitler) et DE-B-1 220 559 (Pfefferkorn). On obtenait bien une fragmentation des gouttelettes liquides en particules sèches de 1/10 à 1/100 de microns mais l'expérience a montré que la qualité des produits obtenus ne pouvait être maintenue constante pendant une longue période, principalement en raison des difficultés d'assurer un débit regulier de la substance à disperser. En particulier la présence de la moindre impureté à l'ajutage de sortie du liquide entraînait l'arrêt ou une alimentation défectueuse du liquide.

L'arrivée irrégulière du liquide entraînait en outre des projections de gouttes de liquides d'une certaine grosseur qui finalement ne pouvaient plus se disperser au contact de la plaque chauffante de sorte que l'on aboutissait à un arrêt de la caléfaction. La plaque se recouvrait d'une couche résiduelle avec parfois une émission d'oxyde de carbone. Ce phénomène pouvait aussi être provoqué par les chutes de la tension alimentant le moteur d'injection d'air, les gouttes de liquide non dispersées contribuant à entraîner le refroidissement de la plaque. De plus l'écoulement des gouttes du liquide hors de la plaque était d'autant plus gènant que le produit avait un caractère acide.

Enfin ces appareils ne pouvaient émettre un brouillard humide de la substance choisie, notamment lorsque celle-ci, sensible à la chaleur, rendait impossible sa projection sur la plaque chauffante.

On connaissait de même des appareils tel que celui décrit au brevet DE-A-2 711 060 (Paul Ritzau Pariwerk) dans lequel les particules du produit étaient projetées de bas en haut sur un élément chauffant à proximité du moyen d'aspiration du produit utilisé. Cependant l'amélioration dûe au renversement du sens des particules ne permettait évidemment d'obtenir que des particules sèches.

Le brevet DE-B-1 046 264 décrit aussi un appareil où les particules d'un produit sont projetées vers le haut au moyen d'un venturi alimenté par un gicleur dont le règlage par rapport à celui-ci permet aux particules d'atteindre ou non une cloche d'arrêt des particules, de telle sorte que l'on peut utiliser le fin brouillard ayant échappé aux parois de la cloche. Bien que ce brouillard soit disponible à une tubulure des parois de l'appareil entourant les parois de la cloche, un tel appareil ne peut évidemment pas produire des particules sèches et le contrôle de l'émission d'un brouillard stable reste toujours très difficile à réaliser.

L'objet de l'invention est un procédé de production d'aérosols à particules sèches ainsi que de brouillards humides caractérisé en ce que l'alimentation des particules liquides s'effectue à partir d'un venturi dont le flux d'air d'entraînement est dirigé verticalement en direction d'un caléfacteur de hauteur réglable et à température réglable.

L'expérience montre alors qu'un tel procédé convient aussi bien à la production d'aérosols de particules sèches de dimension variant entre 1/10 et 1/100 de microns que de brouillard de fines particules humides pour un même réglage initial du venturi et un même débit de l'air d'entraînement du liquide selon le réglage de la température et de la hauteur du caléfacteur.

En effet, l'expérience montre que pour une hauteur suffisante du caléfacteur les particules les plus fines atteignent seules le caléfacteur et produisent des particules aérosols sèches et très fines.

Par contre si l'on diminue la température du caléfacteur jusqu'à la température ambiante le caléfacteur joue, selon sa distance au gicleur et selon le liquide utilisé le rôle de condenseur pour les gouttes de grandes dimensions. Ces gouttes retournent alors au réservoir sans entraver la pulvérisation les petites gouttes se répandant dans l'air.

Un autre objet de l'invention est un appareil de mise en oeuvre de ce procédé qui comprend essentiellement un réservoir de liquide à transformer, un venturi alimenté en air par une source quelconque et alimenté en liquide par un orifice en contact direct avec le liquide du réservoir, un caléfacteur à l'aplomb du venturi, un moyen de réglage de la température du caléfacteur et un moyen de réglage de sa distance au venturi.

Une telle disposition a l'avantage de maintenir un débit régulier du fluide en dépit des petites variations du debit d'alimentation d'air et de recycler impérativement aussi bien les gouttelettes n'atteignant pas le caléfacteur lorsque celui-ci est chauffé que les gouttes de condensation se formant sur le caléfacteur lorsque celui-ci n'étant plus chauffé ne sert qu'à la production d'une pulvérisation humide.

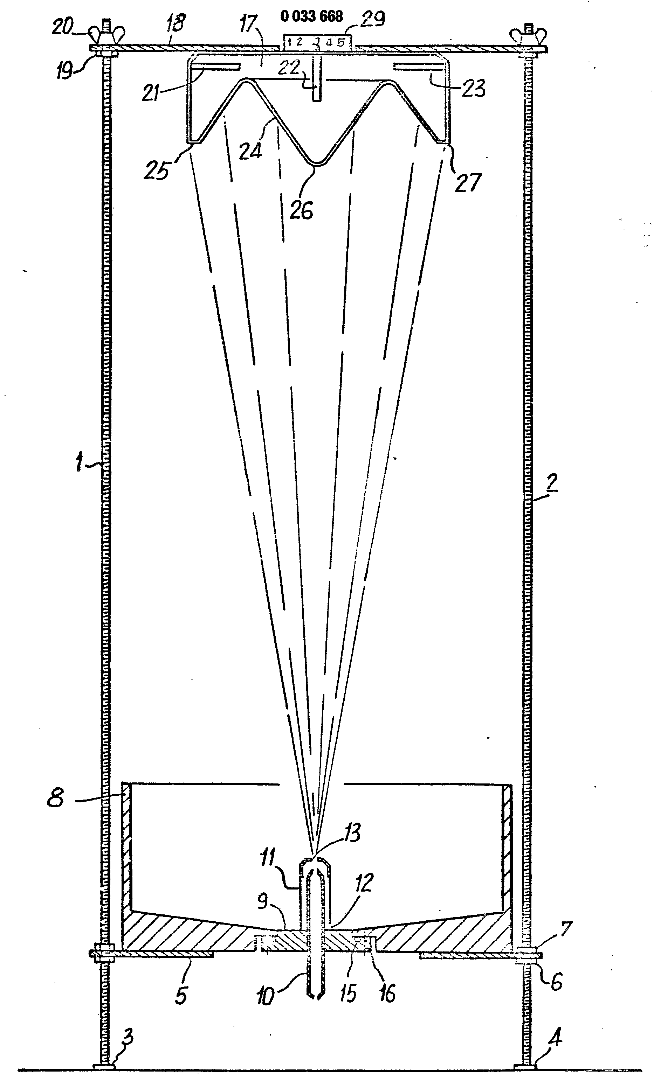

D'autres caractéristiques ressortiront de la description suivante faite en référence au dessin annexé dont la figure unique représente sous forme schématique une coupe en élévation de l'appareil.

Dans le mode de réalisation choisi à titre d'exemple l'appareil est monté sur quatre tiges filetées telles que les tiges représentées en 1 et 2 comportant à leur extrémité inférieure des pieds 3 et 4 pouvant être de tout type.

Ces tiges servent de montants pour la fixation d'une plaque inférieure 5 en forme de couronne comportant à sa périphérie des trous pour le passage des montant tels que 1 et 2, des écrous 6 et 7 assurant la fixation de la plaque à la hauteur voulue sur les montants filetés.

La plaque 5 peut être indépendante du réservoir 8 ou faire corps avec celui-ci. Il peut comporter une partie 9 amovible ou fixe constituant à la fois le fond du réservoir et le support d'un gicleur 10 dont la partie située à l'intérieur du réservoir est coiffée d'une tubulure 11 portant à sa base un orifice 12 pour le passage du liquide et à sa partie supérieure la sortie 13 du venturi. La partie de la périphérie du fond du réservoir est inclinée vers le centre afin de faciliter l'alimentation du venturi lorsque la dose du liquide à traiter s'épuise. Lorsque la partie 9 portant le gicleur 10 est amovible, elle comporte en plus de tout moyen de fixation usuel des joints convenables qui ont été représentés symboliquement en 15 au voisinage des moyens quelconques de fixation 16.

La caléfacteur 17 est monté à l'amplomb de la sortie 13 du venturi qui est réglé pour qu'en position de hauteur maximum du support 18 le caléfacteur 17 soit entièrement intercepté par le cône de liquide pulvérisé provenant de l'orifice 13 sans que la surface extrême du cône de projection puisse atteindre directement la plaque 18.

La plaque 18 peut être réglée en hauteur au moyen de trous de passage des tiges filetées 1 et 2 et d'écrous 19 et 20; ces écrous pouvant être à ailettes afin de faciliter le réglage de la hauteur. On a représenté de façon schématique des résistances de chauffage en 21, 22 et 23 destinées à assurer une répartition convenable de l'apport de chaleur à la plaque 17 en fonction de sa forme. Sa surface, présente en coupe un profil 24 comportant des séries de prolongements 25, 26, 27 facilitant l'écoulement des gouttelettes lorsque celles-ci se condensent sur cette surface en absence d'apport de chaleur. Les gouttelettes ainsi condensées retournent par gravité dans le réservoir.

Un thermostat 29 permet de régler la température du caléfacteur 17.

Ayant réglé une fois pour toute le débit de l'appareil et connaissant la dimension des particules à obtenir on déplace le support 18 en conséquence. S'il s'agit d'obtenir des aérosols constitués de particules sèches comprises entre $1/10$ et $1/100$ de microns on règle la plaque 18 à une hauteur maximum et l'on règle la température du caléfacteur à la valeur correspondant au produit utilisé. Si l'on désire des particules sèches de plus gros diamètre on diminue la distance du caléfacteur 17 à l'orifice 13 et éventuellement la température. L'expérience montre que l'on peut utiliser l'appareil pendant une très longue durée sans encrassage du caléfacteur et quelle que soit sa position et sa température. De même en réglant le thermostat à la valeur de la température ambiante on peut obtenir des pulvérisations humides d'autant plus fines que le support 18 est éloigné de l'orifice 13 du venturi. Comme dans le cas de la production de particules sèches l'appareil peut être utilisé pendant des périodes très longues sans aucun inconvénient même lorsque l'on utilise des solutions acides, les gouttelettes insuffisamment pulvérisées retombant toujours dans le réservoir 8.

Bien que l'on ait décrit qu'un seul mode de réalisation de l'appareil de mise en oeuvre du procédé on comprendra que tous les appareils en dérivant par substitution de moyens équivalents entrent dans le cadre général de l'invention, le prolongement en forme de cône 26 pouvant être plus ou moins effilé en fonction des produits et les métaux utilises pour la caléfacteur étant choisis pour résister aux vapeurs acides.

**Revendications**

1. Procédé de production d'aérosols à particules sèches ainsi que de brouillards humides au moyen d'un venturi alimenté par un flux d'air d'entraînement d'un liquide, ce flux étant dirigé verticalement de bas en haut sur une paroi caléfactrice (17) de séparation des particules liquides et le réglage initial du venturi et du débit d'air d'entraînement alimentant le venturi restant constant, caractérisé en ce que la production d'aérosols à particules sèches de dimensions données, ou de brouillards humides s'effectue par réglage de la hauteur du caléfacteur (17) et de sa température.

2. Appareil de mise en oeuvre du procédé revendiqué en 1 du type comprenant un réservoir (8) du liquide à pulvériser et un venturi central pourvu d'un gicleur (10), ce dernier étant alimenté en air par une source externe, le flux d'air étant dirigé verticalement de bas en haut, et le venturi étant alimenté en liquide par un orifice (12) communiquant avec le niveau du fond incliné du réservoir (8) caractérisé en ce qu'il comporte un moyen de réglage de la hauteur du caléfacteur (17) entre une position basse lui permettant de jouer le rôle de condenseur pour les gouttes de grandes dimensions et une position haute où seules les particules les plus fines l'atteignent et un thermostat (29) de réglage de la température du caléfacteur (17).

3. Appareil tel que revendiqué en 2 dont le gicleur (10) et le venturi sont portés par une pièce cylindrique (9) à la base du réservoir de liquide (8) caractérisé en ce que ladite pièce (9) est montée de façon amovible sur le bord circulaire central du fond incliné du réservoir (8), ledit gicleur (10) étant entouré latéralement, y compris son extrémité conique, d'une tubulure cylindrique coaxiale (11) dont la partie supérieure constitue, avec l'extrémité supérieure du gicleur (10), la sortie (13) d'un venturi et dont la base portée par ladite pièce (9) comporte un orifice (12) mettant en communication directe le fond du réservoir (8) et la base de l'espace annulaire compris entre le gicleur (10) et la tubulure (11).

4. Appareil tel que revendique dans l'une quelconque des revendications 2 et 3 dont le réservoir (8) et le caléfacteur (17) sont montés sur des plaques (5) de niveau réglable.

5. Appareil tel que rendiqué dans l'une quelconque des revendications 2 à 4 dont le caléfacteur comporte des résistances de chauffage (21, 22, 23) disposées dans celui-ci de façon à assurer une répartition convenable d'apport de la chaleur à la plaque contrôlée par le thermostat (29).

6. Appareil tel que revendiqué en 5 dont la surface du caléfacteur (17) comporte des prolongements (25, 26, 27) favorisant l'écoulement des gouttelettes de liquide qui s'y condensent.

7. Appareil tel que revendiqué dans l'une quelconque des revendications 2 à 4 dont les moyens supportant le caléfacteur (17) et le réservoir (8) sont des tiges filetées (1, 2) sur lesquelles se fixent ces éléments au moyen d'écrous (6, 7, 19, 20).

**Patentansprüche**

1. Verfahren zur Erzeugung von Aerosolen von Trockenteilchen sowie von feuchten Nebeln mittels einer durch einen Lufttreibstrahl zum Antrieb einer Flüssigkeit beaufschlagten Venturieinrichtung, wobei der Treibstrahl vertikal von unten nach oben auf eine Heizwand (17) für die Trennung der Flüssigkeitsteilchen geleitet wird und die Anfangseinstellung der Venturieinrichtung und der Durchsatzmenge der die Venturieinrichtung beaufschlagenden Antriebsluft konstant bleibt, dadurch gekennzeichnet, daß die Erzeugung des Aerosols von Trockenpartikeln gegebener Größe oder von feuchten Nebeln durch Einstellung der Höhe der Heizeinrichtung (17) und ihrer Temperatur erfolgt.

2. Vorrichtung zum Durchführen des Verfahrens nach Anspruch 1, mit einem Behälter (8) für die zu zerstäubende Flüssigkeit und einer zentralen, mit einer Düse (10) versehenen Venturieinrichtung welche Düse durch eine externe Quelle mit Luft beaufschlagt wird, wobei der Luftstrom vertikal von unten nach oben gerichtet ist und die Venturieinrichtung über eine auf dem Niveau des geneigten Bodens des Behälters (8) mündende Öffnung (12) mit Flüssigkeit beaufschlagt wird, dadurch gekennzeichnet, daß sie ein Mittel zur Einstellung der Höhe der Heizeinrichtung (17) zwischen einer unteren Position, in der sie als Kondensator für die großen Tröpfchen wirkt und einer oberen Position, wo sie nur von den feinsten Partikeln erreicht wird, und einen Thermostat (29) zur Regelung der Temperatur der Heizeinrichtung (17) aufweist.

3. Vorrichtung nach Anspruch 2, deren Düse (10) und Venturieinrichtung von einem am Boden des Flüssigkeitsbehälters (8) angeordneten zylindrischen Teil (9) getragen werden, dadurch gekennzeichnet, daß das genannte Teil (9) abnehmbar auf dem kreisrunden inneren Rand des geneigten Bodens des Behälters (8) angeordnet ist, daß die Düse (10) einschließlich ihres kronischen Endes seitlich von einem zylindrischen koaxialen Rohrstutzen (11) umgeben ist, dessen oberes Teil mit dem oberen Ende der Düse (10) den Ausgang (13) einer Venturieinrichtung darstellt und dessen von dem genannten Teil (9) getragene Basis eine Öffnung (12) enthält, die die direkte Verbindung zwischen dem Grunde des Behälters (8) und der Basis des ringförmigen Zwischenraums zwischen der Düse (10) und dem Rohrstutzen (11) herstellt.

4. Vorrichtung nach einem der Ansprüche 2 und 3, deren Behälter (8) und deren Heizeinrichtung (17) auf in ihrer Höhe einstellbaren Platten (5) angeordnet sind.

5. Vorrichtung nach einem der Ansprüche 2 bis 4, dadurch gekennzeichnet, daß die Heizeinrichtung Heizwiderstände (21, 22, 23) aufweist, die in ihr so angeordnet sind, daß eine geeignete Verteilung der durch den Thermostat (29) gesteuerten Wärmezufuhr zur Platte gewährleistet ist.

6. Vorrichtung nach Anspruch 5, dadurch gekennzeichnet, daß die Fläche der Heizeinrichtung (17) Fortsätze (25, 26, 27) aufweist, die das Abfließen der dort kondensierenden Flüssigkeitströpfchen begünstigen.

7. Vorrichtung nach einem der Ansprüche 2 bis 4, bei der die die Heizeinrichtung (17) und den Behälter (8) tragenden Mittel Gewindestangen (1, 2) sind, auf denen diese Elemente mit Hilfe von Muttern (6, 7, 19, 20) befestigt sind.

**Claims**

1. A method for producing dry particle aerosols as well as wet mists by means of a venturi fed by an air flux driving a liquid, said flux being directed vertically from bottom to top on a calefactory wall (17) separating the liquid particles, and the initial setting of the venturi and the driving air flow rate feeding the venturi remaining constant, wherein the production of aerosols of dry particle of given sizes, or of wet mists, is provided by setting the height of the calefactor (17) and its temperature.

2. An apparatus for practicing the method as claimed in claim 1, of the type comprising a tank (8) for the liquid to be sprayed and a central venturi provided with a nozzle (10), said nozzle being fred with air from an outer source, the air flux being directed vertically from bottom to top and the venturi being fed with liquid via a port (12) in communication with the level of the inclined bottom of the tank (8), characterized in that it comprises a setting means of the height of the calefactor (17) between a low position allowing it to play the part of a condensor for the large size drops and a high position reached only by the finer particles, and a thermostat (29) for setting the temperature of the calefactor (17).

3. An apparatus as claimed in claim 2, wherein the nozzle (10) and the venturi are supported by a cylindrical part (9) at the base of the liquid tank (8), characterized in that said part (9) is removably mounted on the central circular edge of the inclined bottom of tank (8), said nozzle (10) being surrounded laterally, including its conical end, by a coaxial cylindrical tubing (11) the upper portion of which forms, with the upper end of the nozzle (10) the outlet (13) of the venturi and the base of which, which is carried by said part (9), being formed with a port (12) setting in direct communication the bottom of tank (8) and the base of the annular space enclosed between the nozzle (10) and the tubing (11).

4. An apparatus as claimed in any one of claims 2 and 3, wherein the tank (8) and the calefactor (17) are mounted on plates (5) the level of which is settable.

5. An apparatus as claimed in any one of claims 2 to 4, wherein the calefactor comprises heating resistances (21, 22, 23) arranged in said calefactor such as to provide a convenient distribution of the heat conveyed to the plate controlled by the thermostat (29).

6. An apparatus as claimed in claim 5, wherein the surface of the calefactor (17) is formed with extensions (25, 26, 27) assisting the flow of the liquid droplets condensed thereon.

7. An apparatus as claimed in any one of claims 2 to 4, wherein the means supporting the calefactor (17) and the tank (8) are threaded rods (1, 2) on which are fixed said elements by means of nuts (6, 7, 19, 20).